(19) Europäisches Patentamt
European Patent Office
Office européen des brevets

(11) Numéro de publication : **0 021 925**
**B1**

(12) **FASCICULE DE BREVET EUROPÉEN**

(45) Date de publication du fascicule du brevet : **09.06.82**

(51) Int. Cl.³ : **C 07 C 121/75**, C 07 C 51/09, C 07 C 61/40, C 07 B 19/00, C 07 B 29/00

(21) Numéro de dépôt : **80400809.2**

(22) Date de dépôt : **06.06.80**

(54) **Procédé de préparation d'alcools alpha-cyanés optiquement actifs.**

(30) Priorité : **12.06.79 FR 7914977**

(43) Date de publication de la demande : **07.01.81 (Bulletin 81/01)**

(45) Mention de la délivrance du brevet : **09.06.82 Bulletin 82/23**

(84) Etats contractants désignés : **AT BE CH DE FR GB IT LI LU NL SE**

(56) Documents cités :
**FR A 2 067 854**
**FR A 2 352 768**
**FR A 2 362 829**
**FR A 2 375 161**
**FR A 2 396 745**
**FR A 2 421 875**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140 Bondy (FR)**
Inventeur : **Tessier, Jean**
**30, rue Jean Moulin**
**F-94300 Vincennes (FR)**
Inventeur : **Demoute, Jean-Pierre**
**249 bis rue de Rosny**
**F-93100 Montreuil S/Bois (FR)**

(74) Mandataire : **Petranker, Léon et al**
**boîte postale no 9 102, route de Noisy**
**F-93230 Romainville (FR)**

EP 0 021 925 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de préparation d'alcools α-cyanés optiquement actifs

L'invention concerne un procédé de préparation d'alcools α-cyanés optiquement actifs.

L'invention a pour objet un procédé de préparation d'alcools α-cyanés optiquement actifs, de formule générale II :

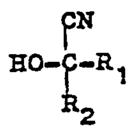

(II)

caractérisé en ce que l'on soumet un ester d'alcool optiquement actif de formule générale (I) :

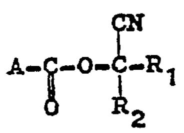

(I)

dans laquelle A représente le reste organique d'un acide chiral carboxylique

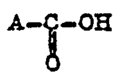

(III)

$R_1$ et $R_2$, représentent soit de l'hydrogène, soit un groupe aliphatique insaturé ou non, substitué ou non, soit un groupe cycloalcanique, aromatique ou hétérocyclique, soit représentent ensemble avec l'atome de carbone auxquels ils sont liés un cycle, l'un au moins des radicaux $R_1$ ou $R_2$ ne représentant pas un atome d'hydrogène, à l'action d'un halogénure de bore, soumet le mélange résultant à l'action de l'eau et obtient l'alcool α-cyané optiquement actif (II) ainsi que l'acide chiral (III), avec rétention totale des configurations absolues de la copule alcoolique et de la copule acide.

L'invention a plus précisément pour objet un procédé de préparation tel que défini précédemment, caractérisé en ce que $R_1$ représente soit un radical alcényle éventuellement substitué, soit un radical alcynyle éventuellement substitué, soit un radical aryle éventuellement substitué par un atome d'halogène ou par un radical méthyle et obligatoirement substitué par un reste arylthio ou aryloxy monocyclique, ces derniers restes pouvant éventuellement être eux-mêmes substitués, soit un radical aryle monocyclique éventuellement substitué par un atome d'halogène, par un radical alcoyle, par un radical alcényloxy, alcynyloxy, aralcoyloxy, phénoxyphényle, benzylfuryle, phénylthiophényle, halophényle, halophénoxyphényle, soit un radical aryle monocyclique substitué par un groupement :

$$—O—(CH_2)_n—C\equiv CH$$

dans lequel n est un entier de 1 à 3 ou par un groupement :

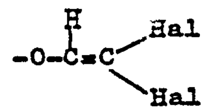

dans lequel Hal représente un atome d'halogène, soit un radical pyrrolyle éventuellement substitué, soit un radical furyle ou thiényle éventuellement substitué par un alcoyle, un alcoyloxyle, un alcynyle, un benzyle, un thiényle, un furylméthyle, un halogène, soit un radical pyridinyle substitué par un reste aryloxy ou arylthio monocyclique, le reste aryloxy ou arylthio étant éventuellement substitué par un alcoyle, un alcoxyle, un alcoylthio, un alcoylsulfonyle, un halogène, soit un radical cycloalcoyle éventuellement substitué, notamment par un groupe —C=O, soit un groupement

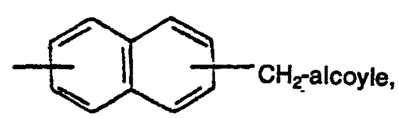

soit un groupement phtalimidométhyle éventuellement substitué, soit un groupement tétrahydrophtalimidométhyle éventuellement substitué et $R_2$ représente un atome d'hydrogène ou un groupement :

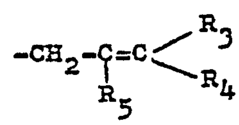

dans lequel, $R_3$, $R_4$, $R_5$ représentent un atome d'hydrogène, un atome de chlore ou un radical méthyle.

L'invention a également pour objet un procédé de préparation tel que défini précédemment, caractérisé en ce que le reste d'acide chiral A représente le reste d'un acide aliphatique, d'un acide comportant un ou plusieurs cycles aromatiques, d'un acide de structure polycyclanique, d'un acide de structure stéroïde ou d'un acide cyclopropane carboxylique.

L'invention a plus particulièrement pour objet un procédé de préparation tel que défini précédemment, caractérisé en ce que l'acide carboxylique est choisi dans le groupe constitué par :

— les acides 2,2-diméthyl 3-(2,2-dibromovinyl)-cyclopropane-1-carboxyliques ;

— les acides 2,2-diméthyl 3-(2,2-dichlorovinyl)-cyclopropane-1-carboxyliques ;

— les acides 2,2-diméthyl 3-(cyclobutylidène méthyl)cyclopropane-1-carboxyliques ;

— les acides 2,2-diméthyl 3-(fluorénylidène méthyl)cyclopropane-1-carboxyliques ;

— les acides 2,2-diméthyl 3-(2-oxo 3-oxa cyclo-

pentylidène méthyl)-cyclopropane-1-carboxyliques ;

— les acides 2,2-diméthyl 3-(2,2-difluorovinyl)-cyclopropane-1-carboxyliques ;

— les acides 2-(3-chlorophényl)2-isopropyl acétiques.

Lorsqu'on utilise les acides carboxyliques nommément cités précédemment, on utilise de préférence comme halogénure de bore, le tribromure de bore ou le trichlorure de bore.

La réaction est alors effectuée notamment dans le chlorure de méthylène à $-20\,°C \pm 20\,°C$.

Dans le procédé selon l'invention, on utilise de préférence 2 moles ou plus d'halogénure de bore par mole d'ester I.

Bien que le mécanisme réactionnel ne soit pas encore entièrement élucidé, il est certain que l'action de l'eau, après la réaction avec l'halogénure de bore, est indispensable pour mener à bien la réaction et obtenir l'alcool α-cyané optiquement actif et l'acide chiral.

Il n'existait, jusqu'ici, qu'un seul procédé de préparation d'alcools (S). α-cyanés, à savoir celui de l'alcool (S) α-cyano 3-phénoxybenzylique, car ces alcools α-cyanés particulièrement fragiles ne pouvaient pas être obtenus à partir de l'alcool (RS) correspondant à l'aide des méthodes connues de dédoublement.

Ce procédé de préparation de l'alcool (S) α-cyano 3-phénoxy benzylique est décrit dans la demande de brevet français publiée sous le n° 2.421.875.

La société demanderesse a maintenant mis au point un procédé de préparation d'alcools α-cyanés, optiquement actifs, à l'état pur, avec un très bon rendement, par une réaction totalement différente de la suite de réactions, indiquée dans ladite demande.

L'utilité du procédé de l'invention réside, notamment, en ce que les alcools α-cyanés optiquement actifs et notamment l'alcool (S) α-cyano 3-phénoxy benzylique, permettent de préparer, par condensation avec des acides convenables, des esters insecticides inaccessibles lorsque l'on ne dispose pas d'alcool (S).

Les acides répondant à la formule III choisis dans le groupe constitué par :

— les acides 2,2-diméthyl 3-(2,2-dibromovinyl)-cyclopropane-1-carboxyliques ;

— les acides 2,2-diméthyl 3-(2,2-dichlorovinyl)-cyclopropane-1-carboxyliques ;

— les acides 2,2-diméthyl 3-(cyclobutylidène méthyl)cyclopropane-1-carboxyliques ;

— les acides 2,2-diméthyl 3-(fluorénylidène méthyl)cyclopropane-1-carboxyliques ;

— les acides 2,2-diméthyl 3-(2-oxo 3-oxa cyclopentylidène méthyl)cyclopropane-1-carboxyliques ;

— les acides 2,2-diméthyl 3-(2,2-difluorovinyl)-cyclopropane-1-carboxyliques ;

— les acides 2-(3-chlorophényl)2-isopropyl acétiques, sont particulièrement intéressants pour être utilisés pour la préparation des produits I.

En effet, les esters d'alcools α-cyanés optiquement actifs de ces acides sont préparés avec un très bon rendement à partir des esters d'alcools (RS) correspondants, en utilisant une base et un solvant insolubilisant de l'ester d'alcool (S) (voir par exemple brevet belge 853.867).

Les exemples suivants illustrent l'invention sans la limiter.

Exemple 1 : Alcool (S) α-cyano 3-phénoxy benzylique

Dans 20 $cm^3$ de chlorure de méthylène, on introduit 2 g de 1R,cis 2,2-diméthyl 3-(2,2-dibromovinyl)cyclopropane-1-carboxylate de (S) α-cyano 3-phénoxy benzyle, refroidit à −40 °C, ajoute, goutte à goutte, à −35 °C, 10 $cm^3$ de solution molaire de tribromure de bore dans le chlorure de méthylène, agite pendant deux heures environ à −30 °C, verse dans un mélange d'eau et de glace sous agitation, amène à pH 7 avec du bicarbonate de sodium, extrait au chlorure de méthylène, concentre à sec par distillation sous pression réduite, chromatographie le résidu sur gel de silice en éluant avec un mélange de benzène et d'acétate d'éthyle (92,5/7,5) et obtient 0,640 g d'alcool (S) α-cyano 3-phénoxy benzylique $[\alpha]_D = -19°$ (c = 0,8 %, chloroforme).

Exemple 2 : Alcool (S) α-cyano 3-phénoxy benzylique

Dans 30 $cm^3$ de chlorure de méthylène, on introduit 3 g de 1R,cis 2,2-diméthyl 3-(2,2-dibromovinyl)cyclopropane-1-carboxylate de (S) α-cyano 3-phénoxy benzyle, refroidit à −40 °C, ajoute, goutte à goutte, à − 35 °C, 15 $cm^3$ de solution molaire de trichlorure de bore dans le chlorure de méthylène, agite pendant deux heures environ à −30 °C, verse dans un mélange d'eau et de glace, sous agitation, amène à pH 7 avec du bicarbonate de sodium, extrait au chlorure de méthylène, concentre à sec sous pression réduite, chromatographie le résidu sur gel de silice en éluant au mélange benzène-acétate d'éthyle (92,5/7,5) et obtient 0,95 g d'alcool (S) α-cyano 3-phénoxy benzylique.

Exemple 3 : Alcool (S) α-cyano 3-phénoxy benzylique

Dans 385 $cm^3$ de chlorure de méthylène, on dissout 38,5 g de 1R,cis 2,2-diméthyl 3-(2,2-dichlorovinyl)cyclopropane-1-carboxylate de (S) α-cyano 3-phénoxy benzyle, amène la température à −35 °C, ajoute lentement 250 $cm^3$ d'une solution 1,6M de trichlorure de bore dans le chlorure de méthylène, agite pendant 30 minutes, verse le mélange réactionnel sur un mélange d'eau et de glace, agite fortement pendant 15 heures, amène à pH 7 par addition de bicarbonate de sodium, extrait au chlorure de méthylène, sèche, concentre à sec sous pression réduite, chromatographie le résidu sur gel de silice en éluant avec un mélange de benzène et d'acétate d'éthyle (92,5/7,5) et obtient 16,9 g d'alcool (S) α-cyano 3-phénoxy benzylique.

Exemple 4 : Alcool (S) α-cyano 3-phénoxy benzylique

Dans 510 $cm^3$ de chlorure de méthylène, on dissout 50,9 g de 1R,cis 2,2-diméthyl 3-(fluorénylidène méthyl)cyclopropane-1-carboxylate de (S) α-cyano 3-phénoxy benzyle, amène la température à −30 °C, ajoute lentement 250 $cm^3$ de solution 1,6M de trichlorure de bore dans le chlorure de méthylène, agite pendant deux heures à −30 °C, verse le mélange réactionnel sur un mélange d'eau et de glace, agite fortement pendant 15 heures, amène à pH 7 par addition de bicarbonate de sodium, extrait au chlorure de méthylène, sèche, concentre à sec sous pression réduite, chromatographie le résidu sur gel de silice en éluant avec un mélange de benzène et d'acétate d'éthyle (92,5/7,5) et obtient 9 g d'alcool (S) α-cyano 3-phénoxy benzylique.

Exemple 5 : Alcool (S) α-cyano 3-phénoxy benzylique

Dans 257 $cm^3$ de chlorure de méthylène, on dissout 25,7 g de 2-(3-chlorophényl)2-isopropyl acétate de (S) α-cyano 3-phénoxy benzyle, amène la température à −5 °C, ajoute lentement 250 $cm^3$ de solution 1,6M de trichlorure de bore dans le chlorure de méthylène, agite pendant deux heures à −5 °C, effectue les mêmes traitements qu'à l'exemple 1, chromatographie le résidu sur gel de silice en éluant avec un mélange de benzène et d'acétate d'éthyle (92,5/7,5) et obtient 11,8 g d'alcool (S) α-cyano 3-phénoxy benzylique.

Exemple 6 : Alcool (S) α-cyano 3-phénoxy benzylique

Dans 345 $cm^3$ de chlorure de méthylène, on dissout 34,5 g de 1R,cis 2,2-diméthyl 3-(cyclobutylidène méthyl)cyclopropane-1-carboxylate de (S) α-cyano 3-phénoxy benzyle, amène la température à −35 °C, ajoute lentement 250 $cm^3$ de solution 1,6M de trichlorure de bore dans le chlorure de méthylène, agite pendant 10 minutes à −35 °C, effectue les mêmes traitements qu'à l'exemple 1, chromatographie le résidu sur gel de silice en éluant avec un mélange de benzène et d'acétate d'éthyle (92,5/7,5) et obtient 18 g d'alcool (S) α-cyano 3-phénoxy benzylique.

**Revendications**

1. Procédé de préparation d'alcools α-cyanés optiquement actifs de formule générale II :

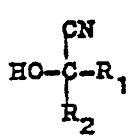

(II)

caractérisé en ce que l'on soumet un ester d'alcool optiquement actif de formule générale I :

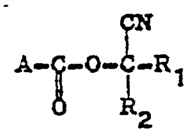

(I)

dans laquelle A représente le reste organique d'un acide chiral carboxylique

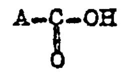

(III)

$R_1$ et $R_2$ représentent soit de l'hydrogène, soit un groupe aliphatique insaturé ou non, substitué ou non, soit un groupe cycloalcanique, aromatique ou hétérocyclique, soit représentent ensemble avec l'atome de carbone auxquels ils sont liés un cycle, l'un au moins des radicaux $R_1$ ou $R_2$ ne représentant pas un atome d'hydrogène, à l'action d'un halogénure de bore, soumet le mélange résultant à l'action de l'eau et obtient l'alcool α-cyané optiquement actif (II) ainsi que l'acide chiral (III), avec rétention totale des configurations absolues de la copule alcoolique et de la copule acide.

2. Procédé de préparation d'alcools α-cyanés, selon la revendication 1, caractérisé en ce que $R_1$ représente soit un radical alcényle éventuellement substitué, soit un radical alcynyle éventuellement substitué, soit un radical aryle éventuellement substitué par un atome d'halogène ou par un radical méthyle et obligatoirement substitué par un reste arylthio ou aryloxy monocyclique, ces derniers restes pouvant éventuellement être eux-mêmes substitués, soit un radical aryle monocyclique éventuellement substitué par un atome d'halogène, par un radical alcoyle, par un radical alcényloxy, alcynyloxy, aralcoyloxy, phénoxyphényle, benzylfuryle, phénylthio phényle, halophényle, halophénoxy phényle, soit un radical aryle monocyclique substitué par un groupement :

$$-O-(CH_2)_n-C\equiv CH$$

ou par un groupement :

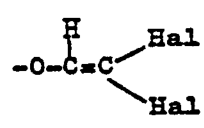

dans lequel Hal représente un atome d'halogène, soit un radical pyrrolyle éventuellement substitué, soit un radical furyle ou thiényle éventuellement substitué par un alcoyle, un alcoyloxy, un alcynyle, un benzyle, un thiényle, un furyl méthyle, un halogène, soit un radical pyridinyle substitué par un reste aryloxy ou arylthio monocyclique, le reste aryloxy ou arylthio étant éventuellement substitué par un alcoyle, un alcoxyle, un alcoyl-

thio, un alcoylsulfonyle, un halogène, soit un radical cycloalcoyle éventuellement substitué, notamment par un groupe —C=0, soit un groupement

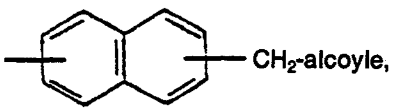

soit un groupement phtalimidométhyle éventuellement substitué, soit un groupement tétrahydrophtalimido méthyle éventuellement substitué et $R_2$ représente un atome d'hydrogène ou un groupement :

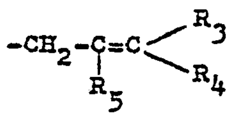

dans lequel $R_3$, $R_4$, $R_5$ représentent un atome d'hydrogène, un atome de chlore ou un radical méthyle.

3. Procédé de préparation selon la revendication 1, caractérisé en ce que le reste d'acide chiral A représente le reste d'un acide aliphatique, d'un acide comportant un ou plusieurs cycles aromatiques, d'un acide de structure polycyclanique, d'un acide de structure stéroïde ou d'un acide cyclopropane carboxylique.

4. Procédé de préparation selon la revendication 3, caractérisé en ce que l'acide carboxylique est choisi dans le groupe constitué par :

— les acides 2,2-diméthyl 3-(2,2-dibromovinyl)-cyclopropane-1-carboxyliques ;

— les acides 2,2-diméthyl 3-(2,2-dichlorovinyl)-cyclopropane-1-carboxyliques ;

— les acides 2,2-diméthyl 3-(cyclobutylidène méthyl)cyclopropane-1-carboxyliques ;

— les acides 2,2-diméthyl 3-(fluorénylidène méthyl)cyclopropane-1-carboxyliques ;

— les acides 2,2-diméthyl 3-(2-oxo 3-oxa cyclopentylidène méthyl)cyclopropane-1-carboxyliques,

— les acides 2,2-diméthyl 3-(2,2-difluorovinyl)-cyclopropane-1-carboxyliques ;

— les acides 2-(3-chlorophényl)2-isopropyl acétiques.

5. Procédé de préparation selon la revendication 4, caractérisé en ce que l'halogénure de bore est le tribromure de bore.

6. Procédé selon la revendication 4, caractérisé en ce que l'halogénure de bore est le trichlorure de bore.

7. Procédé selon la revendication 4, caractérisé en ce que la réaction avec l'halogénure de bore est effectuée dans le chlorure de méthylène à −20 °C ± 20 °C.

8. Procédé selon l'une quelconque des revendications 1 à 7, caractérisé en ce que l'on utilise 2 moles ou plus d'halogénure de bore par mole d'ester I.

**Claims**

1. Process for preparing optically-active α-cyano alcohols of general formula (II) :

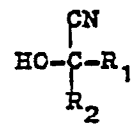 (II)

characterised in that an ester of an optically-active alcohol, of general formula (I) :

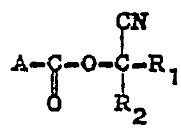 (I)

in which A represents the organic residue of a chiral carboxylic acid

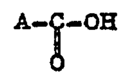 (III)

and

$R_1$ and $R_2$ represent either hydrogen, or a substituted or unsubstituted, unsaturated or saturated aliphatic group, or a cycloalkane, aromatic or heterocyclic group, or represent, together with the carbon atom to which they are bonded, a ring, one at least of the radicals $R_1$ or $R_2$ not representing a hydrogen atom, is subjected to the action of a boron halide, the resulting mixture is subjected to the action of water and the optically-active α-cyano alcohol (II) is obtained as well as the chiral acid (III), with total retention of the absolute configurations of the alcohol moiety and of the acid moiety.

2. Process for preparing α-cyano alcohols, according to Claim 1, characterised in that $R_1$ represents either a possibly-substituted alkenyl radical, or a possibly-substituted alkynyl radical, or an aryl radical possibly substituted by a halogen atom or by a methyl radical and of necessity substituted by a monocyclic arylthio or aryloxy residue, the latter residues being capable possibly of being themselves substituted, or a monocyclic aryl radical possibly susbtituted by a halogen atom, by an alkyl radical, or by an alkenyloxy, alkynyloxy, aralkyloxy, phenoxyphenyl, benzylfuryl, phenylthiophenyl, halophenyl or halophenoxyphenyl radical, or a monocyclic aryl radical substituted by a group :

$$-O-(CH_2)_n-C\equiv CH$$

or by a group :

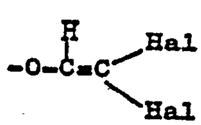

in which Hal represents a halogen atom, or a possibly-substituted pyrrolyl radical, or a furyl or thienyl radical possibly substituted by an alkyl, an alkyloxy, an alkynyl, a benzyl, a thienyl, a furylmethyl or a halogen, or a pyridinyl radical substituted by a monocyclic aryloxy or arylthio residue, the aryloxy or arylthio residue being possibly substituted by an alkyl, an alkoxyl, an alkylthio, an alkylsulphonyl or a halogen, or a cycloalkyl radical possibly substituted especially by a group —C=O, or a group

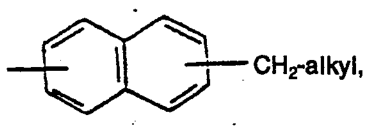

or a possibly-substituted phthalimidomethyl group, or a possibly-substituted tetrahydrophthalimidomethyl group and $R_2$ represents a hydrogen atom or a group :

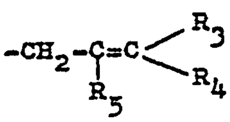

in which $R_3$, $R_4$ and $R_5$ represent a hydrogen atom, a chlorine atom or a methyl radical.

3. Preparation process according to Claim 1, characterised in that the chiral acid residue A represents the residue of an aliphatic acid, of an acid containing one or more aromatic rings, of an acid of polycyclanic structure, of an acid of steroid structure or of a cyclopropane carboxylic acid.

4. Preparation process according to Claim 3, characterised in that the carboxylic acid is selected from the group constituted by :

— the 2,2-dimethyl-3-(2,2-dibromovinyl)-cyclopropane-1-carboxylic acids ;

— the 2,2-dimethyl-3-(2,2-dichlorovinyl)-cyclopropane-1-carboxylic acids ;

— the 2,2-dimethyl-3-(cyclobutylidene-methyl)-cyclopropane-1-carboxylic acids ;

— the 2,2-dimethyl-3-(fluorenylidene-methyl)-cyclopropane-1-carboxylic acids ;

— the 2,2-dimethyl-3-(2-oxo-3-oxa-cyclopentylidene-methyl)-cyclopropane-1-carboxylic acids ;

— the 2,2-dimethyl-3-(2,2-difluorovinyl)-cyclopropane-1-carboxylic acids ; and

— the 2-(3-chlorophenyl)-2-isopropyl-acetic acids.

5. Preparation process according to Claim 4, characterised in that the boron halide is boron tribromide.

6. Process according to Claim 4, characterised in that the boron halide is boron trichloride.

7. Process according to Claim 4, characterised in that the reaction with the boron halide is carried out in methylene chloride at −20 °C ± 20 °C.

8. Process according to any one of Claims 1 to 7, characterised in that two or more moles of boron halide are used per mole of ester I.

**Ansprüche**

1. Verfahren zur Herstellung von optisch aktiven α-cyanierten Alkoholen der allgemeinen Formel II :

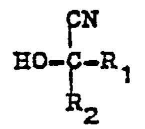
(II)

dadurch gekennzeichnet, daß man einen Ester des optisch aktiven Alkohols der allgemeinen Formel I :

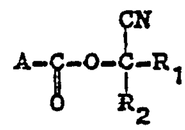
(I)

worin A den organischen Rest einer chiralen Carbonsäure

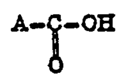
(III)

bedeutet,

$R^1$ und $R^2$ entweder Wasserstoff oder eine ungesättigte oder nicht ungesättigte, substituierte oder nicht substituierte aliphatische Gruppe oder eine Cycloalkangruppe, aromatische oder heterocyclische Gruppe bedeuten oder gemeinsam mit dem Kohlenstoffatom, an das sie gebunden sind, einen Ring bilden, wobei zumindest einer der Reste $R_1$ oder $R_2$ kein Wasserstoffatom darstellt, der Einwirkung eines Borhalogenids unterzieht, das erhaltene Reaktionsgemisch der Einwirkung von Wasser unterzieht und den optisch aktiven α-cyanierten Alkohol (II) sowie die chirale Säure (III) unter vollständiger Beibehaltung der absoluten Konfigurationen der alkoholischen Verknüpfung und der sauren Verknüpfung gewinnt.

2. Verfahren zur Herstellung von α-cyanierten Alkoholen gemäß Anspruch 1, dadurch gekennzeichnet, daß $R_1$ entweder einen gegebenenfalls substituierten Alkenylrest oder einen gegebenenfalls substituierten Alkinylrest oder einen gegebenenfalls durch ein Halogenatom oder durch einen Methylrest und obligatorisch durch einen monocyclischen Arylthio- oder Aryloxyrest substituierten Arylrest, wobei diese letztgenannten Reste gegebenenfalls ihrerseits substituiert sein können, oder einen gegebenenfalls durch ein Halogenatom, durch einen Alkylrest, durch einen Alkenyloxy-, Alkinyloxy-, Aralkyloxy-, Phenoxyphenyl-, Benzylfuryl-, Phenylthiophenyl-, Halophenyl-, Halophenoxyphenylrest substituierten monocyclischen Arylrest oder einen monocyclischen Arylrest, substituiert durch eine Gruppe :

$$-O-(CH_2)_n-C\equiv CH$$

worin n eine ganze Zahl von 1 bis 3 bedeutet, oder durch eine Gruppe :

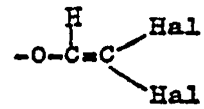

worin Hal ein Halogenatom bedeutet, oder einen gegebenenfalls substituierten Pyrrolylrest oder einen gegebenenfalls durch einen Alkyl-, einen Alkoxy-, einen Alkinyl-, einen Benzyl-, einen Thienyl-, einen Furylmethylrest oder ein Halogenatom substituierten Furyl- oder Thienylrest oder einen durch einen monocyclischen Aryloxy- oder Arylthiorest substituierten Pyridinylrest, wobei der Aryloxy- oder Arylthiorest gegebenenfalls durch einen Alkyl-, einen Alkoxy-, einen Alkylthio-, einen Alkylsulfonylrest oder ein Halogenatom substituiert sein kann, oder einen gegebenenfalls substituierten, insbesondere durch eine Gruppe $-C=O$ substituierten Cycloalkylrest oder eine Gruppe

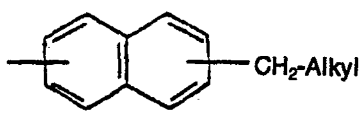

oder eine gegebenenfalls substituierte Phthalimidomethylgruppe oder eine gegebenenfalls substituierte Tetrahydrophthalimidomethylgruppe bedeutet und $R_2$ ein Wasserstoffatom oder eine Gruppe

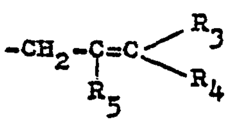

bedeutet, worin $R_3$, $R_4$ und $R_5$ ein Wasserstoffatom, ein Chloratom oder einen Methylrest darstellen.

3. Verfahren gemäß Anspruch 1, dadurch gekennzeichnet, daß der Rest der chiralen Säure A der Rest einer aliphatischen Säure, einer Säure mit einem oder mehreren aromatischen Ringen, einer Säure mit Polycyclan-Struktur, einer Säure mit Steroid-Struktur oder einer Cyclopropancarbonsäure ist.

4. Verfahren gemäß Anspruch 3, dadurch gekennzeichnet, daß die Carbonsäure ausgewählt wird unter :

— den 2,2-Dimethyl-3-(2,2-dibromvinyl)-cyclopropan-1-carbonsäuren,

— den 2,2-Dimethyl-3-(2,2-dichlorvinyl)-cyclopropan-1-carbonsäuren,

— den 2,2-Dimethyl-3-(cyclobutylidenmethyl)-cyclopropan-1-carbonsäuren,

— den 2,2-Dimethyl-3-(fluorenylidenmethyl)-cyclopropan-1-carbonsäuren,

— den 2,2-Dimethyl-3-(2-oxo-3-oxa-cyclopentylidenmethyl)-cyclopropan-1-carbonsäuren,

— den 2,2-Dimethyl-3-(2,2-difluorvinyl)-cyclopropan-1-carbonsäuren,

— den 2-(3-Chlorphenyl)-2-isopropylessigsäuren.

5. Verfahren gemäß Anspruch 4, dadurch gekennzeichnet, daß das Borhalogenid das Bortribromid ist.

6. Verfahren gemäß Anspruch 4, dadurch gekennzeichnet, daß das Borhalogenid das Bortrichlorid ist.

7. Verfahren gemäß Anspruch 4, dadurch gekennzeichnet, daß die Umsetzung mit dem Borhalogenid in Methylenchlorid bei $-20\,°C \pm 20\,°C$ durchgeführt wird.

8. Verfahren gemäß einem der Ansprüche 1 bis 7, dadurch gekennzeichnet, daß man 2 Mol oder mehr Borhalogenid je Mol Ester I verwendet.